(19) **Europäisches Patentamt / European Patent Office / Office européen des brevets**

(11) **EP 1 867 659 A1**

(12) **EUROPEAN PATENT APPLICATION**
published in accordance with Art. 158(3) EPC

(43) Date of publication:
**19.12.2007 Bulletin 2007/51**

(21) Application number: **06730854.4**

(22) Date of filing: **31.03.2006**

(51) Int Cl.:
**C07K 16/18** (2006.01)   **C07K 16/44** (2006.01)
**C07K 1/22** (2006.01)   **C12P 21/08** (2006.01)
**G01N 33/53** (2006.01)   **G01N 33/577** (2006.01)

(86) International application number:
**PCT/JP2006/306906**

(87) International publication number:
**WO 2006/109599 (19.10.2006 Gazette 2006/42)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **05.04.2005 JP 2005108623**

(71) Applicant: **JMS Co., Ltd.**
**Hiroshima-shi (JP)**

(72) Inventors:
• **YAMAMOTO, Takashi**
  **Hiroshima-shi,**
  **Hiroshima 731-3168 (JP)**
• **KIMURA, Yuko**
  **Hiroshima-shi, Hiroshima 730-0051 (JP)**

(74) Representative: **Behnisch, Werner**
**Reinhard-Skuhra-Weise**
**Friedrichstrasse 31**
**80801 München (DE)**

(54) **ANTIBODY REACTIVE SPECIFICALLY TO AGE DERIVED FROM 3,4-DGE**

(57) The present invention provides antibodies against AGEs derived from carbonyl compounds that are highly reactive with proteins or peptides, and methods of detecting the AGEs derived from the carbonyl compounds. 3,4-dideoxyglucosone-3-ene (3,4-DGE) is allowed to react with proteins, a host animal is immunized with the reaction product thereof, AGEs, and antibodies against the AGEs (anti-AGE antibodies) are isolated from serum recovered from the host animal. These anti-AGE antibodies thus isolated are allowed to react with a sample, and then the antigen-antibody reaction between the AGEs in the sample and the anti-AGE antibodies is detected. Thereby the presence or amount of the AGEs in the sample can be detected.

(1)3,4-DGE-BSA   (9)3-DG-BSA
(2)BSA   (10)5-HMF-BSA
(3)3,4-DGE-RSA   (11)Fur-BSA
(4)RSA   (12)AA-BSA
(5)3,4-DGE-HAS   (13)FA-BSA
(6)HAS   (14)glycer-BSA
(7)MGO-BSA   (15)glycol-BSA
(8)GO-BSA   (16)Glu-BSA

(1) (2) (3) (4) (5) (6) (7) (8) (9) (10) (11) (12) (13) (14) (15) (16)

Lane No.

Fig.3

## Description

Technical Field

The present invention relates to antibodies against advanced glycation endproducts (AGEs) and methods of detecting AGEs using the same.

Background Art

A protein glycation reaction (Maillard reaction) is a nonenzymatic reaction between amino groups of amino acids, peptides, or proteins and ketones or aldehydes (particularly reducing sugars). The protein glycation reaction can be divided into two reactions that occur in the early stage and the later stage. The reaction in the early stage is a reversible reaction. In the early stage, for example, amino groups and reducing sugars react with each other to form Schiff bases, and subsequently Amadori compounds are formed through an intramolecular rearrangement reaction. On the other hand, the reaction in the later stage is an irreversible reaction. In the later stage, the Amadori compounds further are subjected to complicated reaction processes such as rearrangement and condensation and thereby stable substances that are referred to as "advanced glycation endproducts (AGEs)" are formed. Examples known as the AGEs include carboxymethyllysine (CML), pentosidine, pyrraline, crossline, etc. However, it is considered that various unknown AGEs whose structures have not been identified yet exist in vivo.

Recently, products of the aforementioned Maillard reaction are attracting attention in medical fields. The relationship between the products and various illness, diseases, or aging has been reported. Particularly, it has been known that known AGEs not only deteriorate the functions of proteins but also induce cytotoxicity and inflammatory responses. Furthermore, it also has been considered that the formation of AGEs causes proteins to aggregate and to be insolubilized to be accumulated abnormally in tissues, and this denatured the tissues. Moreover, typical examples of known protein glycation reactions in vivo include an increase in hemoglobin A1C (the Amadori compounds formed by the reaction in the early stage) in diabetics, and accumulation ofAGEs in arteriosclerotic lesion sites and kidneys subject to chronic renal failure and diabetic nephropathy.

For the measurement of the aforementioned Maillard reaction products, immunological assays have been known in addition to high performance liquid chromatography and gas chromatography. Particularly, immunological detection methods, specifically, for instance, an immunohistological method and an enzyme immunoassay are used widely for researches and clinical diagnoses in the medical field because they are simple and require no special analyzers, for example. Accordingly, various antibodies that react specifically with glycated proteins or AGEs have been developed. Specifically, antibodies against CML, pentosidine, crossline, and pyrraline have been produced, and immunological studies with respect to tissues of animals with diseases such as aging, diabetes, nephropathy, etc. have been reported (for instance, Nonpatent Document 1, Nonpatent Document 2, Nonpatent Document 3, Nonpatent Document 4, and Nonpatent Document 5). Furthermore, the method of using anti-CML antibodies as diabetes markers (Patent Document 1), and monoclonal antibodies against Nδ-(5-hydroxy-4,6-dimethylpyrimidine-2-yl)-ornithine that is an AGE (Patent Document 2) also have been reported.

It has been confirmed that reducing sugars are decomposed and thereby carbonyl compounds such as 3-deoxyglucosone (3-DG), methylglyoxal, glyoxal, etc. are produced in metabolic decomposition of glucide, pyrolysis of reducing sugars, etc. in vivo or in the early stage of the Maillard reaction. It has been reported that these 3-DG, methylglyoxal, and glyoxal are highly reactive with proteins and are potent mediators (hereinafter also referred to as "AGE precursors") that are involved in AGE formation as described above. Since a large amount of AGEs derived from those carbonyl compounds exist in the blood of patients on hemodialysis, it is suggested that the AGEs have relevance to dialysis complications (Nonpatent Document 6 and Nonpatent Document 7).

As described above, decomposition products of reducing sugars have been attracting attention as causative substances of AGE formation and the studies thereof are proceeding. However, the decomposition pathway of reducing sugars is complicated and it has been known that a wide variety of carbonyl compounds are produced thereby, but it has not been clarified what type of carbonyl compounds actually is involved in AGE formation, and many ofAGEs that are derived from such carbonyl compounds have not been denatured. Hence, it has been common to substitute AGEs whose structures have been elucidated, particularly CML, in the studies ofAGEs including quantification of AGEs.

Nonpatent Document 1: Schleicher, E. D. et al., J. Clin. Invest. 99, 457, 1997
Nonpatent Document 2: Sanaka, T. et al., Nephron 91, 64, 2002
Nonpatent Document 3: Obayashi, H. et al., Biochem. Biophys. Res Commun. 226,37,1996
Nonpatent Document 4: Miyata, S. and Monnier, V, J. Clin. Invest. 89, 1102, 1992
Nonpatent Document 5: Hayase, F. et al., J. Biol. Chem. 263, 3758, 1989
Nonpatent Document 6: TAKEUCHI, Masayoshi et al., Nippon Rinsho 60, Extra Number 8, 2002, Nippon Rinsho
Nonpatent Document 7: Takeuchi, M. et al., Molecular Medicine 7, 783, 2001
Patent Document 1: JP9(1997)-178740A
Patent Document 2: JP11(1999)-246599A

Disclosure of Invention

Problem to be Solved by the Invention

Hence, in the medical field, there is demand for a method of analyzing/measuring new AGEs that accumulate in vivo, have biological activity, and can cause various illness and diseases.

Accordingly, the present invention is intended to provide antibodies against AGEs that are derived from carbonyl compounds by specifying the carbonyl compounds that are highly reactive with proteins or peptides, i.e. have high AGE formation ability. Furthermore, the present invention is intended to provide methods of detecting the AGEs using the antibodies.

Means for Solving the Problem

An antibody of the present invention is an antibody against an advanced glycation endproduct (AGE). The antibody of the present invention is characterized in that the AGE is a reaction product of 3,4-dideoxyglucosone-3-ene (3,4-DGE) and a protein or peptide.

Effect of the Invention

As a result of keen studies made assiduously, the present inventors found out that the carbonyl compound (3,4-DGE) produced from glucose had a very high reactivity to proteins and a great effect on biological functions as compared to known AGE precursors (i.e. carbonyl compounds that were causative substances of forming AGEs from proteins, for example). Based on this finding, antibodies against reaction products of the 3,4-DGE and proteins or peptides were developed and thus the present invention was completed. As described above, the antibodies of the present invention are antibodies that specifically recognize the reaction products of 3,4-DGE and proteins, for example. Accordingly, it is possible to detect efficiently 3,4-DGE-derived AGEs that are considered to have a great effect on biological functions. Hence, it is considered that the antibodies of the present invention are useful for diagnoses and medical treatments of various diseases, such as those described later, in which conjecturally the 3,4-DGE-derived AGEs are involved.

Brief Description of Drawings

FIG. 1 is a graph for determining an association constant of an anti-3,4-DGE-RSA antibody in an example of the present invention.

FIGs. 2(A) and 2(B) are graphs showing the reaction specificity of the anti-3,4-DGE-RSA antibody in another example of the present invention.

FIG. 3 is a photograph showing the result of Western blotting using the anti-3,4-DGE-RSA antibody in further another example of the present invention.

FIG. 4 shows photographs indicating the results obtained by exposing human peritoneal mesothelial cells to 3,4-DGE and then allowing them to undergo an antigen-antibody reaction with the anti-3,4-DGE-RSA antibody in still another example of the present invention; FIG. 4(a) shows a control, while FIGs. 4 (b) and 4(c) show the results of the example.

FIG. 5 shows photographs indicating the results obtained by exposing a rat peritoneal cavity to a dialysate and then allowing this to undergo an antigen-antibody reaction with the anti-3,4-DGE-RSA antibody in yet another example of the present invention; FIG. 5(A) shows a control, while FIGs. 5(B) and 5(C) show the results obtained when 2 $\mu$M of 3,4-DGE-containing dialysate and 58 $\mu$M of 3,4-DGE-containing dialysate were used, respectively.

FIGs. 6(A) and 6(B) are graphs showing the reaction specificity of an anti-3,4-DGE-RSA antibody in further another example of the present invention.

FIG. 7 is a graph for determining the association constant of the anti-3,4-DGE-RSA antibody in the above-mentioned example.

FIG. 8 is a graph showing the reactivity between an anti-3,4-DGE-RS antibody and AGE-proteins that are different from each other in time of the reaction between 3,4-DGE and a protein in still another example of the present invention.

FIG. 9 is a photograph showing coloration of reaction solutions of 3,4-DGE and a protein in a reference example of the present invention.

FIG. 10 is a graph showing a time-dependent change in fluorescence intensity during the reaction between 3,4-DGE and a protein in the aforementioned reference example.

FIG. 11 shows graphs indicating time-dependent changes in residual ratios of Arg residues and Lys residues in a reaction product of 3,4-DGE and a protein in the above-mentioned reference example; FIG. 11(A) shows the residual ratio of Arg residues, while FIG. 11(B) shows the residual ratio of Lys residues.

FIG. 12 is a graph showing a time-dependent change in isoelectric point of the reaction product of 3,4-DGE and a protein in the above-mentioned reference example.

FIG. 13 shows graphs indicating time-dependent changes in SDS-PAGE of the reaction product of 3,4-DGE and a protein in the above-mentioned reference example; FIG. 13(A) shows the result obtained in using 3,4-DGE, while FIG. 13(B) shows the result obtained using MGO.

FIG. 14 is a graph showing the reactivities to proteins of various carbonyl compounds in another reference example of the present invention.

FIG. 15 is a graph showing cytotoxicity of the products of various carbonyl compounds and proteins in still another reference example of the present invention.

FIG. 16 is a photograph showing the results of Western blotting of the anti-3,4-DGE-RSA antibody in further another example of the present invention.

FIG. 17 is a photograph showing the results of Western blotting of the anti-3,4-DGE-RSA antibody in still another example of the present invention.

Best Mode for Carrying Out the Invention

The anti-AGE antibodies of the present invention are antibodies against reaction products, AGE, of 3,4-DGE and proteins or peptides as described above.

Preferably, the anti-AGE antibodies of the present invention do not react with the following: AGEs derived from carbonyl compounds such as methylglyoxal (MGO), glyoxal (GO), 3-deoxyglucosone (3-DG), 5-hydroxymethyl-furfural (5-HMF), furfural (Fur), formaldehyde (FA), glucose (Glu), acetaldehyde (AA), etc. that are known AGE precursors, particularly reaction products of the carbonyl compounds and proteins or peptides. Furthermore, it also is preferable that the anti-AGE antibodies of the present invention do not react with proteins or peptides that have at least one of a pentosidine residue and a carboxymethyllysine (CML) residue that are known AGEs, for example.

An example of the method of preparing anti-AGE antibodies of the present invention is described below.

(1) Preparation of AGEs to Serve as Antigen (Immunogen)

AGEs are prepared by allowing 3,4-DGE and proteins (or peptides; the same applies below) to undergo a Maillard reaction. As described later in Reference Example 1, 3,4-DGE has a very high reactivity to proteins as compared to known AGE precursors such as methylglyoxal (MGO), glyoxal (GO), 3-deoxyglucosone (3-DG), etc. that are involved in AGE formation. Hence, when 3,4-DGE and proteins are mixed together and then are incubated, 3,4-DGE allows AGEs to be formed from the proteins and thus reaction products, AGEs, are obtained.

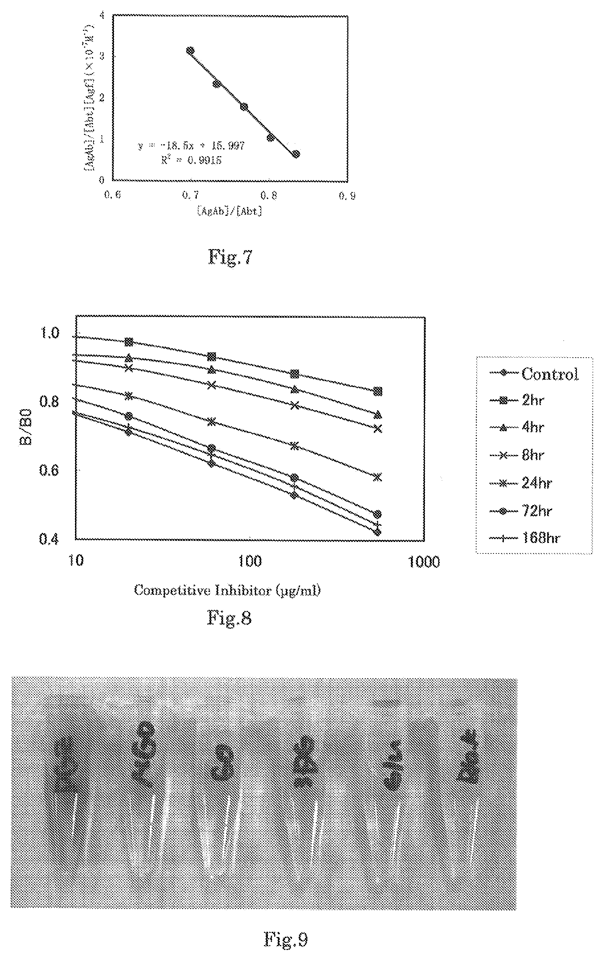

Generally, whether AGEs have been formed or not can be judged depending on whether the reaction solution of 3,4-DGE and proteins is brown. In addition, the progress thereof can be judged by a change in brown intensity. The browning of the reaction solution is described later in a reference example (see FIG. 9). Furthermore, a reaction product generates fluorescence when 3,4-DGE allows AGEs to be formed from proteins. Accordingly, it also can be judged by the fluorescence intensity, for example. The conditions for measuring the fluorescence intensity are not particularly limited but include, for example, an excitation wavelength of 320 to 370 nm and a fluorescence wavelength of 400 to 470 nm, preferably an excitation wavelength of 345 nm and a fluorescence wavelength of 425 nm.

The protein that allows 3,4-DGE to undergo the Maillard reaction is not particularly limited. Examples thereof include albumin such as serum albumin, hemoglobin, myoglobin, hemocyanin, etc. They can be derived from various mammals such as rabbits, cattle, humans, and various birds such as chickens, quails, etc., for example. Specific examples thereof include rabbit serum albumin (RSA), bovine serum albumin (BSA), human serum albumin (HSA), ovalbumin, etc. Furthermore, the peptide can be either natural peptide or synthetic peptide and also can be oligopeptide or polypeptide.

The antibodies that have been prepared allow 3,4-DGE-derived AGEs to be detected regardless of the degree of reaction (for instance, the amount of 3,4-DGE added thereto). In order to form AGEs from all proteins in preparing antigens, for example, it is considered that it is necessary to add 3,4-DGE in at least an equivalent amount to that of $NH_2$ groups of the proteins. For instance, it is preferable that 3,4-DGE be added so that the amount thereof is 0.1 to 100 equivalent to that of the amino groups of the proteins, more preferably 1 to 10 equivalent, and particularly preferably 3 to 5 equivalent. Furthermore, 3,4-DGE can be added to proteins two times or more to allow AGEs to be formed sufficiently

The temperature for incubating the 3,4-DGE and proteins is not particularly limited. For example, it is 25 to 50°C, preferably 35 to 40°C. The time for each incubation also is not particularly limited. For instance, it is 3 to 14 days, preferably 7 to 10 days.

Generally, it is preferable that the 3,4-DGE and proteins be allowed to react with each other in a buffer solution whose pH is for example 6 to 8, preferably 7 to 7.5. The type and concentration of the buffer solution are not particularly limited and can be selected according to the desired pH. Examples of the buffer solution include a phosphate buffer solution, a sodium hydrogen maleate - NaOH buffer solution, etc. Buffer solutions free from amino groups are preferred. The concentration of the buffer solution in the reaction solution also is not particularly limited but is in the range of 10 to 500 mM, for example.

Furthermore, a chelator such as diethylenetriamine pentaacetic acid (DTPA) may be added to the reaction solution. The concentration of the chelator is in the range of 1 to 100 mM, for example.

When 3,4-DGE allows AGEs to be formed from proteins, the reaction products, AGEs, generally generate fluorescence and are browned. Accordingly, whether AGEs have been formed can be checked through visual observation of the reaction products or measurement of fluorescence intensity as described above, for example.

Preferably, in order to desalt or remove low molecular compounds (for instance, unreacted 3,4-DGE, etc.), the reaction products (AGEs) that have been obtained generally are dialyzed and filtrated to be sterilized and then are used as immunogens.

(2) Preparation of Antibody

The method of preparing antibodies is not limited. For instance, polyclonal antibodies and monoclonal antibodies can be prepared by conventionally known processes of producing antibodies by immunizing animals. The type of the host animals to be immunized is not particularly limited. Examples of the host animals that can be used herein include human, mammals other than human, such as rabbit, rat, mouse, goat, sheep, horse, pig, guinea pig, etc., and birds such as chicken, pigeon, duck, quail, etc. The method of administering antigens also is not particularly limited. The method that can be employed is intradermal administration, subcutaneous administration, intraperitoneal administration, intravenous administration, intramuscular administration, etc, preferably subcutaneous administration, intraperitoneal administration, or intravenous administration, and more preferably subcutaneous administration.

For instance, when polyclonal antibodies are to be prepared, the following process can be employed. That is, the aforementioned antigens (AGEs) are administered to a host animal such as the one described above. Thereby the animal is immunized, and thereafter anti-AGE antibodies are isolated from, for example, serum or ascitic fluid that has been collected therefrom and then are purified. On the other hand, when monoclonal antibodies are to be prepared, the following process can be employed. That is, for example, an antibody producing cell such as a spleenocyte or lymphoidocyte of an immunized host animal and a myeloma cell are fused with each other to prepare a hybridoma. The hybridoma is proliferated, hybridoma cells that produce antibodies with specificity are isolated, and thus monoclonal antibodies can be obtained.

The method of purifying polyclonal antibodies or monoclonal antibodies also is not limited. For instance, the purification can be carried out by conventionally known methods such as salting-out, dialysis, ion exchange chromatography, affinity chromatography, electrophoresis, etc.

The method of screening production of target antibodies is not particularly limited and, for example, a conventionally known radioimmunoassay (RIA) or enzyme immunoassay (EIA) method can be employed.

Generally, in the antibodies obtained in this manner, the immunoglobulin class is IgM or IgG. In addition, the antibody molecules obtained thereby also can be used as antibodies per se or active fragments of antibodies such as Fab, Fab', $F(ab')_2$, etc. that are obtained by further enzyme-treating the antibody molecules also can be used as antibodies of the present invention.

Next, the description is directed to a method of detecting the reaction products, AGEs, of 3,4-DGE and proteins (or peptides) using antibodies of the present invention. The present invention provides a method of detecting AGEs in a sample using the antigen-antibody reaction between the AGEs in the sample and anti-AGE antibodies against the AGEs. The antigen-antibody reaction is caused by allowing the sample and the anti-AGE antibodies to react with each other. The method is characterized in that the AGEs are reaction products of 3,4-DGE and proteins or peptides, and the anti-AGE antibodies are anti-AGE antibodies of the present invention.

The use of such a detection method makes it possible to detect AGEs formed with 3,4-DGE in various samples. As described before, 3,4-DGE has a very high reactivity to, for example, proteins, and both 3,4-DGE itself and AGEs formed with 3,4-DGE are highly toxic to cells. Accordingly, it can be understood that when the method of detecting such 3,4-DGE-derived AGEs according to the present invention is used for clinical practice, for example, the method is useful for diagnosing or preventing diseases that are considered to be affected by the 3,4-DGE-derived AGEs. In specific examples, it is surmised that detection of 3,4-DGE-derived AGEs makes it possible to judge the possibility of development, the degree of development, the stage of progression, etc. with respect to: various diseases related to the pathology of renal failure or diabetes; complications of diabetes or renal failure; diseases accompanying aging; complications accompanying peritoneal dialysis, such as peritoneal fibrosis, peritoneal sclerosis, sclerosing encapsulating peritoneal sclerosis, etc.

The antigen-antibody reaction described above can be detected by, for instance, EIA methods (for example, a competitive EIA method and an indirect EIA method), RIA methods, fluoroimmunoassay (FIA), chemiluminescent immunoassay (CLIA), turbidimetric immunoassay (TIA), latex turbidimetric immunoassay (LTIA), or immunoagglutination methods such as a gold colloid particle method. For instance, anti-AGE antibodies and a sample (a sample containing antigen AGEs) are allowed to react with each other and then this reaction solution is added to a carrier to which antigen AGEs have been immobilized beforehand. In this case, antibodies that did not react with the antigens in the sample in the reaction solution are bonded to the immobilized antigens AGEs. Subsequently, the reaction solution is removed from the carrier and then labeled-antibodies (secondary antibodies) against the anti-AGE antibodies are added thereto. This allows the secondary antibodies to bond to the anti-AGE antibodies that have been bonded to the immobilized antigens AGEs, and the labels of the secondary antibodies that eventually have been bonded to the anti-AGE antibodies are detected.

The above-mentioned carrier is not particularly limited. Examples thereof include beads, plates (for instance, immunoplates), tubes, etc. Examples of the label include enzymes such as peroxidase, alkaline phosphatase, etc., fluorescent materials, light-emitting materials, radioisotopes, etc.

Labeling of antibodies can be carried out by conventional methods according to the type of the label. When the label is, for example, an enzyme, a substrate that is colored through an enzymatic reaction may be added and the degree to which the substrate is colored may be measured in terms of absorbance, for example. Furthermore, in the case of a radioisotope, radioactivity may be measured with a scintillation counter, for example. Each of such absorbance, radioactivity, fluorescence intensity, etc. has a relative relationship with the amount of the antibodies that has been bonded to immobilized antigens. Accordingly, the amount of the antibodies can be quantified using a calibration curve that has been prepared beforehand, for example. Furthermore, the amount of the antibodies that has been bonded to the immobilized antigens is the amount of free antibodies that did not react with the antigens in the sample. Hence, the amount of the antibodies that has been bonded to the antigens in the sample can be calculated from the amount of the free antibodies and is equivalent to the amount of the antigens contained in the sample. Thus, the antigen AGEs in the sample also can be quantified. The method of detecting the antigen-antibody reaction is not limited to such methods, and conventionally known methods can be employed.

The sample to be tested, which is used in this detection method, is not particularly limited. Examples thereof include various samples such as serum, blood plasma, blood, urine, body fluids such as spinal fluid, extracts from biological cells, culture solutions for a fungus body, etc. Furthermore, the detection method of the present invention also can be carried out with respect to biological tissues directly.

Next, a method of detecting a carbonyl compound that forms an AGE using an antibody of the present invention is described. The method of detecting a carbonyl compound of the present invention is a method of detecting a carbonyl compound in a sample that forms an AGE, using an anti-AGE antibody against the AGE. This method is characterized as follows. The carbonyl compound is 3,4-DGE, while the AGE is a reaction product of the 3,4-DGE and a protein or peptide. The anti-AGE antibody is an anti-AGE antibody of the present invention. The method includes: allowing the 3,4-DGE in the sample and the protein or peptide to react with each other; allowing a product obtained through the above-mentioned reaction and the anti-AGE antibody to react with each other; detecting an AGE formed through an antigen-antibody reaction between the product and the anti-AGE antibody; and qualitatively or quantitatively determining the 3,4-DGE in the sample from the presence or amount of the AGE.

As described above, both 3,4-DGE itself and AGEs formed with 3,4-DGE are highly toxic to cells. Hence, for example, when 3,4-DGE is contained in, for example, a dialysate, 3,4-DGE-derived AGEs are formed in the biological body to which such a dialysate has been administered, and thereby may affect the biological body. According to the method of detecting 3,4-DGE of the present invention, for example, it is possible to check (qualitatively determine) the presence of 3,4-DGE in a sample such as, for example, the dialysate and to quantify the content thereof. Accordingly, the method makes it possible to evaluate as to whether the dialysate is one with a low risk. Thus the method of detecting 3,4-DGE of the present invention can be said to be a method for judging quality that is very useful in the medical field.

The sample to be tested, for which the method of detecting a carbonyl compound of the present invention is used, is not particularly limited. For example, it can be a dialysate as described above, an intravenous drip, an injection, a foodstuff such as beverage, etc. Particularly, dialysates and intravenous drips generally contain saccharides, and the components thereof may have been changed to substances (AGEs precursors) that are involved in AGE formation, due to the heat treatment for sterilizing. Hence, when the presence of 3,4-DGE that is an AGE precursor is checked beforehand by the detection method of the present invention, safer dialysates, intravenous drips, etc. can be provided for patients.

The method of the present invention can be carried out in the same manner as in the aforementioned method of detecting an AGE of the present invention except that a sample and a protein or peptide are allowed to react with each other beforehand and then an antibody of the present invention is allowed to react with the reaction product of the sample and the protein or peptide. That is, when a sample is allowed to be reacted with a protein, etc. and thereby an antigen-antibody reaction between the reaction product and the antibody of the present invention is observed, it means that 3,4-DGE-derived AGEs have been formed. Accordingly, it can be judged that 3,4-DGE exists in the sample. Furthermore, the content of 3,4-DGE also can be quantified according to the degree of the antigen-antibody reaction.

The protein that is allowed to react with the sample is not particularly limited. Examples thereof include serum albumin and hemoglobin. For instance, when an intravenous drip that is used as a sample is administered, it also is preferable that a protein of a tissue that has a high possibility of coming into contact with the intravenous drip be used. This further makes it possible to predict satisfactorily the AGE formation that occurs when it is administered to a biological body.

Furthermore, an immunoreagent of the present invention is one containing an anti-AGE antibody against an AGE. It is characterized in that the AGE is a reaction product of 3,4-DGE and a protein or peptide, while the anti-AGE antibody is the above-mentioned anti-AGE antibody according to the present invention. The immunoreagent of the present invention can be used in the method of detecting AGEs and the method of detecting 3,4-DGE of the present invention described above. The method for use thereof is the same as in the case of the anti-AGE antibody of the present invention.

In the immunoreagent of the present invention, the anti-AGE antibody can be labeled with various labeling substances depending on the method of detecting the antigen-antibody reaction, for example. Furthermore, as long as the immunoreagent of the present invention contains an anti-AGE antibody of the present invention, it is not limited in composition other than that.

Hereinafter, the present invention is described further in detail using examples and comparative examples but is not limited thereto. The unit "%" denotes "weight%" unless otherwise specified.

EXAMPLE 1

Preparation of Anti-3,4-DGE-Derived AGE Polyclonal Antibody

(1) Preparation of AGE Antigen (3,4-DGE-derived AGEs)

First, 500 mM of 3,4-DGE aqueous solution was prepared. Separately, RSA (10 mg/ml) and DTPA (5 mM) were dissolved in 0.2 M sodium phosphate buffer (PB: pH 7.4). Furthermore, the above-mentioned 3,4-DGE aqueous solution was mixed thereinto in such a manner that the amount of 3,4-DGE was 2.5-equivalent relative to that of $NH_2$ groups in the RSA. This mixed solution was sterilized by filtration with a 0.2-$\mu$m filter and then was incubated at 37°C for three days. Furthermore, the above-mentioned 3,4-DGE aqueous solution was mixed thereinto again in such a manner that the amount of 3,4-DGE was 2.5-equivalent relative to that of $NH_2$ groups in the RSA. Then this was incubated at 37°C for four days. Thereafter, this reaction solution was applied to a desalting column (Trade Name: PD-10, manufactured by Amarsham Biosciences). Then the solution recovered therefrom was dialyzed with PBS(-) throughout the day and night and thereby desalting and removal of low molecular compounds were performed. The above-mentioned PBS is 10 mM phosphate buffer containing 0.15 M sodium chloride. The solution obtained after this dialysis was sterilized by filtration with the 0.2-$\mu$m filter. Thus an antigen (3,4-DGE-derived AGE) solution was obtained. This antigen solution was allowed to have a protein concentration of 7 mg/ml.

(2) Immunization of Rabbit

The antigen solution (with a protein concentration of 7 mg/ml) was mixed with an equivalent amount of complete Freund's adjuvant and thereby was emulsified. This emulsion was administered subcutaneously to several places in a dorsal region of each rabbit biweekly. In this case, the dosage per administration was 5 mg/rabbit in terms of the amount of protein. Blood was collected over time from the start of immunization, and the antibody titer was checked by indirect ELISA. As a result, it was judged that the antibody titer had increased satisfactorily through subcutaneous immunization in the dorsal region that had been carried out five times in total. Hence, in the last (sixth) administration, the original antigen solution described above was administered to the ear vein of the rabbit. Then ten days later, the whole blood was collected from the immunized rabbit, with the rabbit being anesthetized.

(3) Preparation of Anti-3,4-DGE-derived AGE Polyclonal Antibody

(3-1) Separation of Antiserum

The rabbit blood thus obtained was allowed to stand still at room temperature for approximately three hours and thereby blood clot and serum were separated naturally. Thereafter, they were centrifuged (3500 rpm, 10 minutes) and then the supernatant collected therefrom was centrifuged (3500 rpm, 10 minutes) again. The supernatant (antiserum) thus obtained was divided into 10-ml small portions and then they were subjected to an inactivation treatment at 56°C for 30 minutes. They were cryopreserved at -80°C until they were required for use.

(3-2) Purification of Polyclonal Antibody

First, 10 ml of antiserum that had been subjected to the above-mentioned treatment was mixed with an equivalent amount of 0.02 M sodium phosphate buffer (pH 7.0). Thereafter, this was filtrated with a 0.45-$\mu$m filter and then was subjected to IgG affinity chromatography under the conditions mentioned below. Thus the polyclonal antibody was purified.

> Column: Affi-Gel Protein A Agarose for IgG Purification (manufactured by Bio-Rad Laboratories, Inc.)
> Column Size: 10 ml ($\varnothing$ 10 $\times$ 100 mm)
> Eluent: (A) 0.02 M sodium phosphate buffer (pH 7.0)
> (B) 0.1 M glycine - hydrochloric acid buffer (pH 2.7)

Elution Condition: Step gradient from (A) to (B)

Flow Rate: 1 ml/min

First, the aforementioned antiserum was applied to the column that had been equilibrated with the eluent (A) and thereby was eluted with the eluent (A) described above. Thereafter, the absorbance with a wavelength of 280 nm of the eluted fraction was measured successively. When the absorbance of the eluted fraction became approximately zero, the eluent was substituted with the eluent (B). Then the eluted fraction (protein fraction) obtained with the eluent (B) was collected. Then 1 M Tris-HCl buffer (pH 9.0) was added to the fraction thus recovered and thereby neutralized the fraction. Thereafter, this was centrifuged and concentrated until the amount thereof became about 10 ml. This was used as a purified anti-3,4-DGE-derived AGE polyclonal antibody solution (with a protein concentration of 9.7 mg/ml). The above-mentioned antibody was divided into 1-ml small portions and they were cryopreserved at -80°C until they were required for use. With respect to the anti-3,4-DGE-derived AGE polyclonal antibody thus obtained, for example, characteristics thereof were evaluated in Examples 2 to 5. Moreover, polyclonal antibodies were prepared several times according to the method of Example 1. As a result, the similar antibodies were obtained with reproducibility.

Example 2

With respect to the anti-3,4-DGE-derived AGE polyclonal antibody obtained in Example 1, the association constant thereof was determined.

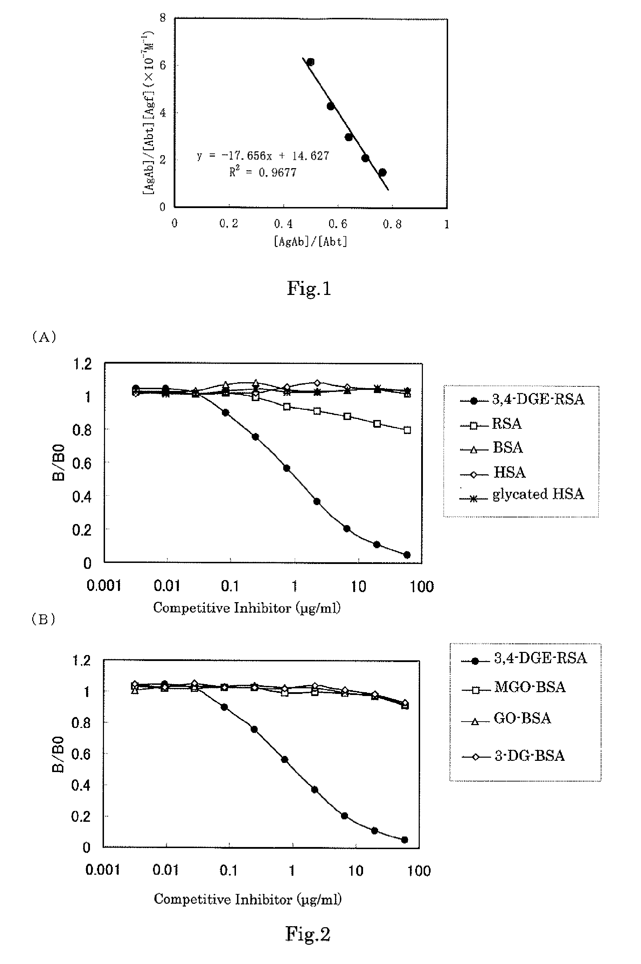

The association constant was determined by competitive ELISA. First, the antigen solution prepared in Example 1 was diluted with 50 mM sodium carbonate buffer so as to be 1 μg/ml. Then 100 μl thereof was added to each well of a 96-well immunoplate and then was incubated at room temperature for two hours. Thus the antigen was immobilized. After the two hours incubation, the antigen solution was removed and then each well was washed with 0.05% Tween 20-containing PBS (TPBS). Thereafter, 300 μl of 0.5% skim milk-containing PBS was added to each well. This was incubated at room temperature for two hours and thereby the portions to which the antigens had not been fixed were blocked. After the two hours incubation, the blocking solution was removed and then each well was washed with TPBS. Thereafter, 50 μl of antigen solutions having various concentrations that had been diluted with 0.1% glycerin and 50 ml Tris-HCl buffer containing 0.1% Tween 20 (pH 7.4: TB) as well as 50 μl of antibody (primary antibody) solution of Example 1 that had been diluted 2500 times with 0.1% skim milk-containing TB were added thereto. This was incubated at room temperature for two hours. After the two hours incubation, the reaction solution was removed and each well was washed with TPBS. Thereafter, 100 μl of solution of alkaline phosphatase-labeled sheep anti-rabbit IgG antibody (a solution obtained by adding and dissolving 1 ml of water and 1 ml of glycerin to a lyophilizate (manufactured by CHEMTCON)) against the above-mentioned primary antibody that had been diluted 2250 times with 0.3% skim milk-containing TB was added thereto. This was incubated at 37°C for one hour. After the one hour incubation, the reaction solution was removed and then each well was washed with TPBS. Thereafter, 100 μl of chromogenic reagent (that was obtained by dissolving 2 ml of Diethanolamine Substrate Buffer (Trade Name) (5x) (manufactured by PIERCE) and two tablets of ImmunoPure PNP PTablets (Trade Name) (manufactured by PIERCE) in 9 ml of water) were added to each well. This was incubated at room temperature for 30 minutes. After the incubation, 50 μl of 2 M sodium hydroxide aqueous solution was added to each well to stop the reaction of alkaline phosphatase. Then the absorbance at 405 nm was measured. Thereafter the graph shown in FIG. 1 was created using the following formula and then the dissociation constant Kd was calculated.

$$Kd = k2 / k1 = [Agf][Abf] / [AgAb]$$

[Agf]: Free Antigen Concentration
[Abf]: Free Antibody Concentration
[AgAb]:Antigen-Antibody Complex Concentration
The dissociation constant Kd determined from FIG. 1 was $5.7 \times 10^{-9}$ (M). Furthermore, since the association constant Ka is the reciprocal of the dissociation constant Kd, it was calculated as $1.8 \times 10^8$ (M$^{-1}$). Since the association constant of a common polyclonal antibody is $10^7$ to $10^9$ (M$^{-1}$), it can be said that the antibody obtained in Example 1 has a sufficiently high strength of association with the antigen.

Example 3

With respect to the anti-3,4-DGE-derivedAGE polyclonal antibody obtained in Example 1, the reaction specificity thereof was evaluated.

The specificity to 3,4-DGE-derived AGE-protein was evaluated by Western blotting and the same competitive ELISA as that used for determining the association constant. The various AGE-proteins described below were prepared in the same manner as in "(1) Preparation ofAntigen" in Example 1. Glu-BSA was prepared by dissolving BSA (10 mg/ml) and DTPA (5 mM) in 0.2 M PB (pH 7.4), adding Glu thereto so that the total amount was 100 mM, and incubating it at 37°C for eight weeks.

(1) Competitive ELISA

The reaction specificity was evaluated by ELISA in the same manner as in Example 2 except for using, as competitive inhibitors, AGE-proteins formed with 3,4-DGE, "3,4-DGE-RSA", native proteins, "RSA, BSA, and HSA", AGE-proteins formed with carbonyl compounds (MGO, GO, and 3-DG) other than 3,4-DGE, "MGO-BSA, GO-BSA, and 3-DG-BSA", and glycated proteins, "glycated HSA (manufactured by SIGMA; Trade Name: A-8301)". The results are shown in FIGs. 2(A) and 2(B). FIG. 2(A) is a graph showing the results obtained using 3,4-DGE-RSA, native proteins, and glycated HSA, while FIG. 2(B) is a graph showing the results obtained using 3,4-DGE-RSA and other AGE-proteins. In FIGs. 2(A) and 2(B), "B" denotes absorbance at 405 nm obtained when the competitive inhibitors were added to the wells, "B0" denotes absorbance at 405 nm obtained when the competitive inhibitors were not added to the wells, and the unit (μg/ml) of the competitive inhibitors denotes the concentration of the competitive inhibitor added to the wells.
As shown in FIG. 2(A), the polyclonal antibody obtained in Example 1 did not cross-react with the native proteins and glycated HSA. Furthermore, as shown in FIG. 2(B), it also did not cross-react with other AGE-proteins.

(2) Western Blotting

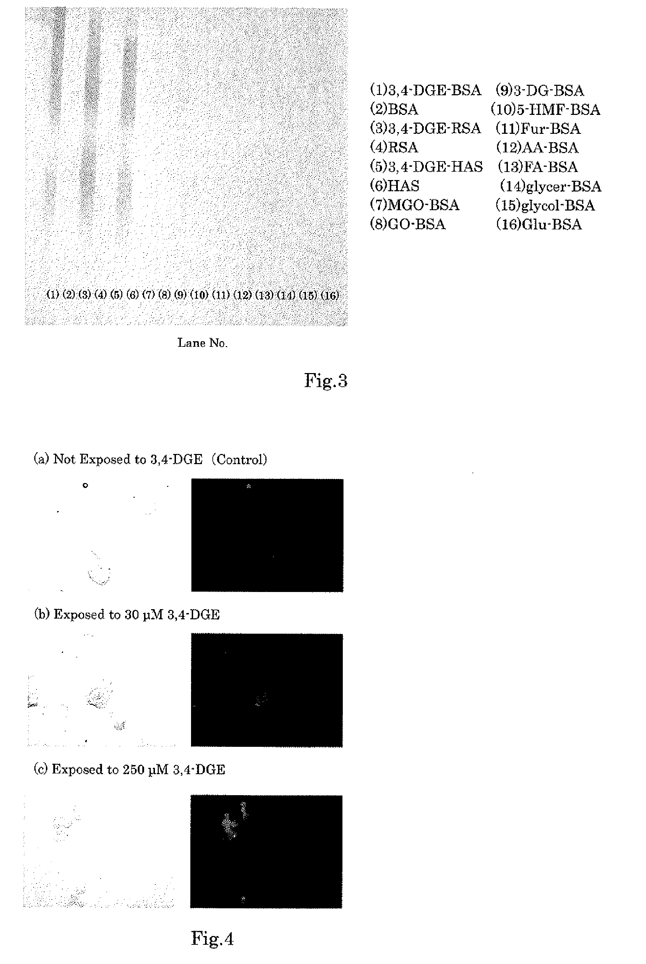

Samples used herein were AGE-proteins formed with 3,4-DGE, "3,4-DGE-RSA, 3,4-DGE-BSA, and 3,4-DGE-HSA", native proteins, "RSA, BSA, and HSA", AGE-proteins formed with Glu and carbonyl compounds (MGO, GO, 3-DG, 5-HMF, Fur, AA, FA, glycer, and glycol) other than 3,4-DGE, "MGO-BSA, GO-BSA, 3-DG-BSA, 5-HMF-BSA, Fur-BSA, AA-BSA, FA-BSA, glycer-BSA, glycol-BSA, and Glu-BSA". These samples each (1 $\mu$g per sample) were subjected to SDS-PAGE and each protein band was blotted onto a polyvinylidene fluoride (PVDF) membrane. This membrane was immersed in 0.3% skim milk-containing TTBS (0.15 M sodium chloride and 25 mM Tris-HCl buffer containing 0.1% Tween 20 (pH 7.4)). Then this was incubated at room temperature for one hour and thereby blocking was carried out. After the incubation, the blocking solution was removed and then the membrane was washed with TTBS. Thereafter, it was immersed in a solution of the antibody (primary antibody) according to Example 1 that had been diluted 12000 times with the blocking solution, and then it was incubated at room temperature for one hour. After the incubation, the primary antibody solution was removed and then the membrane was washed with TTBS. Thereafter, it was immersed in a solution of alkaline phosphatase-labeled sheep anti-rabbit IgG antibody (manufactured by CHEMICON) against the above-mentioned primary antibody that had been diluted 12000 times with the blocking solution. This was incubated at room temperature for one hour. After the incubation, the reaction solution was removed and then the membrane was washed with TTBS. Thereafter, it was allowed to develop color with a chromogenic reagent (BCIP/NBT (nitroblue tetrazolium / 5-bromo-4-chloro-indolyl phosphate); manufactured by Promega) prepared according to the instructions for use. The results are shown in the photograph in FIG. 3. In the description above, "5-HMF" denotes 5-hydroxymethyl-furfural, "Fur" indicates furfural, "AA" denotes acetaldehyde, "FA" indicates formaldehyde, glycer denotes glyceraldehyde, and "glycol" indicates glycolaldehyde.

As shown in FIG. 3, color development was observed only in the 3,4-DGE-BSA (Lane No. 1), 3,4-DGE-RSA (Lane No. 3), and 3,4-DGE-HSA (Lane No. 5) that were AGEs formed with 3,4-DGE. No cross-reaction with the native proteins and AGE-proteins formed with other carbonyl compounds was shown.

From the results of the competitive ELISA and Western blotting described above, it was found out that the polyclonal antibody of Example 1 specifically recognized only AGE-proteins formed with 3,4-DGE.

Example 4

With 3,4-DGE, AGEs were formed from intracellular proteins, and then the AGEs were detected using the polyclonal antibody of Example 1.

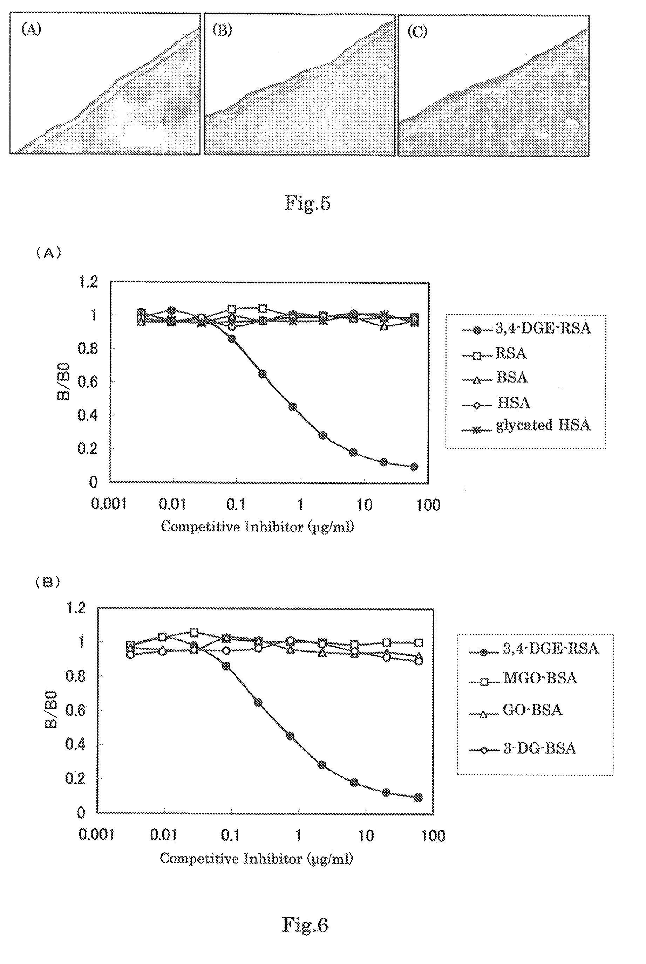

Human peritoneal mesothelial cells (HPMCs) that had been suspended in M199 medium containing 20% FBS (fetal bovine serum) (hereinafter, referred to as "FBS-M199 medium") were seeded into an 8-well slide chamber and were cultured (37°C) until they became confluent. After culturing, the medium was removed from the slide chamber, and then a 3,4-DGE solution, which had been diluted with the M199 medium in such a manner as to be 30 $\mu$M or 250 $\mu$M, was exposed to the HPMCs. For a control, the M199 medium was exposed to the HPMCs. After culturing for two hours, the M199 medium was removed and then the HPMCs were washed with PBS. Further, cold methanol with a temperature of -30°C was poured thereinto, and thereby HPMCs were fixed at -30°C for five minutes. Subsequently, after they were washed with PBS, 0.2% TritonX-100-containing PBS was allowed to drip thereinto and this was allowed to stand at room temperature for five minutes. Thus a penetration treatment was carried out. Then the penetration solution was removed and HPMCs were washed with PBS. Thereafter, PBS containing 5% normal pig serum (manufactured by DAKO) was allowed to drip thereinto and this was allowed to stand at room temperature for five minutes. Thus blocking was carried out. Then the blocking solution was removed. Thereafter, the antibody (primary antibody) solution of Example 1 that had been diluted 500 times with the PBS containing 5% normal pig serum was allowed to drip thereinto. This was incubated at room temperature for one hour. After the incubation, the primary antibody solution was removed and HPMCs were washed with PBS. Subsequently, a solution of FITC-labeled pig anti-rabbit IgG antibody (manufactured by DAKO) against the primary antibody that had been diluted 30 times with the PBS containing 5% normal pig serum was allowed to drip thereinto. This was incubated in a dark place at room temperature for one hour. After the incubation, the reaction solution was removed and HPMCs were washed with PBS. Then they were observed with a fluorescence microscope. The results are shown in photomicrographs in FIG. 4. In FIG. 4, (a) shows the control that was not exposed to 3,4-DGE, (b) shows the result obtained in the case where the 3,4-DGE concentration was 30 $\mu$M, and (c) shows the result obtained in the case where the 3,4-DGE concentration was 250 $\mu$M. In FIG. 4, the photographs shown on the left side are those taken in the bright field, while those shown on the right side are fluorescence photomicrographs.

As shown in FIGs. 4(b) and 4(c), fluorescence was observed only in the HPMCs that had been exposed to the 3,4-DGE. In the control shown in FIG. 4(a), fluorescence was not detected. Accordingly, it was found out that in human cells, the polyclonal antibody of Example 1 was bound specifically only to AGE-proteins formed with 3,4-DGE to which they had been exposed, and that the polyclonal antibody of Example 1 was applicable to antibody staining in human cells.

Example 5

With respect to a model rat for peritoneal dialysis, accumulation of 3,4-DGE-derived AGEs in the peritoneum was examined.

Dialysates in which 3,4-DGE concentrations were 2 μM and 58 μM were administered to the peritoneal cavities of two groups of rats for 30 days (twice/day; 7 rats per group). With respect to a control group (7 rats), no dialysates were administered and a needle stick alone was carried out. The parietal peritoneum of each rat was excised and was freeze-embedded. Then a thin section was produced. This section was fixed with paraformaldehyde. Then a solution of the antibody (primary antibody) according to Example 1 that had been diluted 500 times with 0.5% skim milk-containing PBS was allowed to drip thereonto. This was incubated at room temperature for one hour. After the incubation, the primary antibody solution was removed and then the section was washed with TTBS. Thereafter, it was treated with an alkaline phosphatase-labeled secondary antibody kit (Trade Name: DAKO LSAB 2 System Alkaline Phosphatase; manufactured by DAKO) for the primary antibody according to the instructions for use. After the reaction solution was removed and the section was washed with TTBS, this was allowed to develop color with a chromogenic reagent (Trade Name: New Fuchsin; manufactured by DAKO) that had been prepared according to the instructions for use. The results are shown in photographs in FIG. 5. In FIG. 5, (A) shows the control, (B) is a photograph showing the result obtained from a rat dialyzed with 2 μM 3,4-DGE-containing dialysate, and (C) is a photograph showing the result obtained from a rat dialyzed with 58 μM 3,4-DGE-containing dialysate.

As shown in FIG. 5, when compared to the control (shown in FIG. 5(A)), color development was observed in the rats dialyzed with the 3,4-DGE-containing dialysates (FIGs. 5(B) and 5(C)), particularly, stronger color development was observed in the rat dialyzed with the dialysate containing a larger amount of 3,4-DGE (FIG. 5(C)). Accordingly, it is surmised that the 3,4-DGE contained in the dialysates allowed AGEs to be formed in the peritoneal tissues. Furthermore, from this experiment, it can be said that the polyclonal antibody of Example 1 also can stain antibodies of biological tissues.

Example 6

An anti-3,4-DGE-dorived AGE polyclonal antibody was prepared by the same method as in Example 1 described above and then was compared with the antibody of Example 1 with respect to the reaction specificity and association constant.

The reaction specificity was evaluated by competitive ELISA as in Example 3. The results are shown in FIGs. 6(A) and 6(B). FIG. 6(A) is a graph showing the results obtained using 3,4-DGE-RSA, native proteins, and glycated HSA, while FIG. 6(B) is a graph showing the results obtained using 3,4-DGE-RSA and other AGE-proteins. In FIGs. 6(A) and 6(B), "B" and "B0" have the same meaning as described before.

These results were compared with the results obtained using the antibody prepared in Example 1 (FIGs. 2(A) and 2(B). The polyclonal antibody of Example 6 did not cross-react with the native proteins and glycated HSA and also did not cross-react with the other AGE-proteins as shown in FIG. 6(B). The polyclonal antibody of Example 6 exhibited the same behavior as that of the antibody of Example 1.

The association constant was determined by competitive ELISA in the same manner as in Example 2. It was calculated from the graph shown in FIG. 7, in the same manner as in Example 2. As a result, the association constant determined from FIG. 7 was calculated as $1.9 \times 10^8$ $(M^{-1})$. Thus it was proved that the association constant was comparable to that of the polyclonal antibody of Example 1 calculated in Example 2. From the results described above, it is confirmed that similar antibodies can be obtained with high reproducibility according to the method of Example 1.

Example 7

AGE antigens (3,4-DGE-derived AGEs) that were different from each other in reaction time were prepared. It was evaluated whether they can be detected by the anti-3,4-DGE-derivedAGE polyclonal antibody of Example 1 regardless of the reaction time.

Antigens (3,4-DGE-derived AGEs) were prepared in the same manner as in Example 1 except that BSA (5 mg/ml) was dissolved in PBS (pH 7.4), the aforementioned 3,4-DGE aqueous solution was mixed therewith in such a manner that the amount of 3,4-DGE was 4-equivalent to that of $NH_2$ groups in BSA, and then this was incubated at 37°C for predetermined times (2, 4, 8, 24, 72, and 168 hours). Thereafter, the reactivity between each antigen thus obtained and the polyclonal antibody of Example 1 was evaluated by the same competitive ELISA as in Example 3. The results are shown in the graph in FIG. 8. In FIG. 8, "B" and "B0" have the same meaning as described before.

As shown in FIG. 8, all the results obtained with the respective antigens showed the similar downward-sloping behaviors. Thus, it is understood that the reactivity thereof depends on the competitive concentration. From the results, it can be said that the similar AGEs are formed regardless of the time for which the protein and 3,4-DGE are allowed to react with each other. Since the B/B0 value decreases depending on the reaction time, it can be said that the extent to which AGEs are formed varies depending on the reaction time. It can be surmised that in order to form AGEs from all proteins, incubation is necessary to be carried out for approximately 72 hours, for example.

<Reference Example 1>

With 3,4-DGE, AGEs were formed from proteins, and then the reactivity and physical properties of the AGEs obtained thereby were evaluated.

1. Reactivity to Protein

<Appearance>

AGEs were formed from proteins, BSA, using carbonyl compounds (3,4-DGE, MGO, GO, 3-DG, and Glu). First, BSA (manufactured by SIGMA; Trade Name: A-0281) was dissolved in PBS (pH 7.4) so as to have a concentration of 5 mg/ml (6.2 mM of basic amino acid residues). Thus a BSA solution was prepared. Then each carbonyl compound was added to the BSA solution so as to have a concentration of 25 mM (4-equivalent relative to the basic amino acid residues in the BSA). This was incubated at 37°C for predetermined times (2, 4, 8, 24, 72, and 168 hours). The respective carbonyl compounds used herein were 500 mM 3,4-DGE aqueous solution (prepared in house), 40% MGO aqueous solution (manufactured by SIGMA), 40% GO aqueous solution (manufactured by Wako Pure Chemical Industries, Ltd.), 3-DG (manufactured by Dojindo Laboratories Co., Ltd.), and Glu (Pharmacopeia, manufactured by San-ei Sucrochemical Co, Ltd.). The BSA solution was incubated in the same manner as described above, with no carbonyl compounds being added thereto. This was used as a control. The appearances of the reaction solutions obtained after 168 hours incubation are shown in the photograph in FIG. 9. FIG. 9 shows, from the left, the results obtained using 3,4-DGE (DGE), MGO, GO, 3-DG (3DG), Glu, and Blank (control).

In the case of using 3,4-DGE, the reaction solution was colored in approximately two hours and thereafter became browner over time. As shown in FIG. 9, deep brown was observed 168 hours later. Furthermore, as shown in FIG. 9, browning was not observed in the reaction solution obtained using Glu but was observed in the reaction solutions obtained using MGO, GO, and 3-DG that were known as mediators for AGE formation. Accordingly, it can be said that AGE formation in the later stage of Maillard reaction occurs in the case of using 3,4-DGE as in the case of using MGO, GO, and 3-DG. Furthermore, since the color thereof is deep brown, it also is understood that 3,4-DGE has a strong reactivity to proteins.

<Fluorescence Intensity>

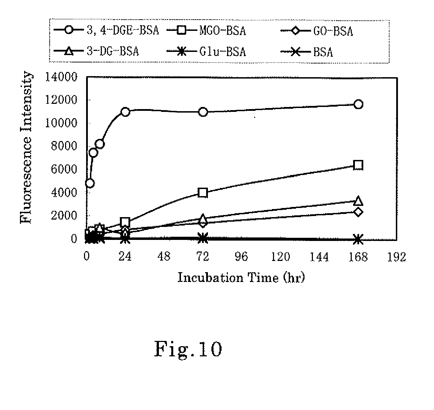

The reaction solutions incubated as described above were sampled and these reaction solutions thus sampled were applied to desalting columns (Trade Name: PD-10, manufactured by Amersham Pharmacia Biotech) to be desalted. With respect to the reaction solutions thus desalted, the BSA concentration was measured using BCA protein assay kit (manufactured by Pierce). Then the reaction solutions were diluted with water so as to have a BSA concentration of 1.0 mg/ml. Thus sample solutions were obtained. These sample solutions were cryopreserved at -30°C until they were required for use. These sample solutions were dispensed into a 96-well white plate. Then the fluorescence intensity was measured at an excitation wavelength of 360 nm and a fluorescence wavelength of 430 nm (measuring apparatus: Trade Name SPECTRAFLUOR PLUS, manufactured by TECAN). The results are shown in FIG. 10. In FIG. 10, "BSA" indicates a control to which no carbonyl compound was added.

As shown in FIG. 10, the sample obtained using 3,4-DGE had very high fluorescence intensity as compared to those obtained using other carbonyl compounds and nearly achieved equilibrium after 24 hours reaction.

2. Evaluation of Reaction Product

With respect to the above-mentioned sample solutions obtained using the carbonyl compounds (3,4-DGE, 3-DG, GO, and MGO), it was evaluated by ELISA whether pentosidine, carboxymethyllysine (CML), and Maillard reaction products (MRX: 8-hydroxy-5-methyldihydrothiazolo [3,2-alpha]) that were known AGEs had been produced or not. As a result, in the reaction solution obtained using GO, production of CML of 3.025 molecules per BSA molecule was found already after 24 hours incubation and production of CML of 3.7 molecules per BSA molecule was found after 168 hours incubation. In the reaction solution obtained using MGO, pentosidine of 0.0028 molecule per BSA molecule was found after 168 hours incubation. On the other hand, in the reaction solution obtained using 3,4-DGE, production of neither CML, pentosidine, or MRX was found. Accordingly, it can be said that the AGEs formed with 3,4-DGE are clearly different from conventionally known AGEs.

3. Amino Acid Analysis

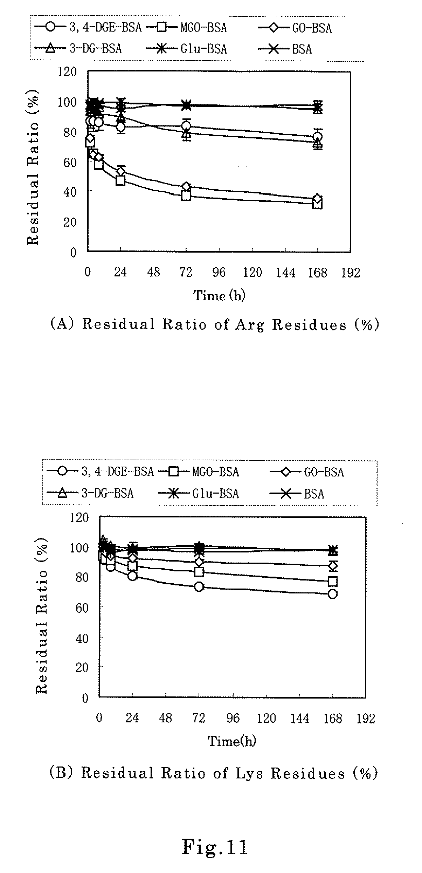

The aforementioned sample solutions were freeze-dried and then were acid-hydrolyzed in 6N hydrochloric acid (120°C, 24 hours). According to a conventionally known method, these were derivatized with dansyl chloride and were isolated with HPLC. Then the amounts of arginine (Arg) and lysine (Lys) residues in the samples were determined (n = 3). The results are shown in FIGs. 11(A) and 11(B). FIG. 11(A) is a graph showing the residual ratios of the Arg residues, while FIG. 11(B) is a graph showing the residual ratios of the Lys residues. The residual ratio indicates a percentage that is obtained with the amount of residues in untreated native BSA being taken as 100%.

As shown in FIG. 11(A), free Arg and Lys were reduced as in the case of AGEs obtained using other carbonyl compounds. Accordingly, it is surmised that 3,4-DGE reacted with the Arg and Lys residues as in the case of conventionally known AGEs.

## 4. Isoelectric Focusing

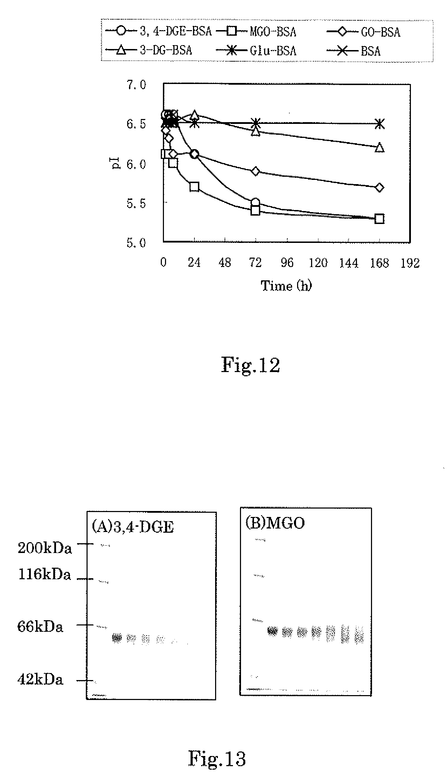

A pI marker was electrophoresed beforehand and then a calibration curve between pI and mobility was created. Then the aforementioned sample solutions also were electrophoresed. With a region stained deepest in each sample being taken as the center of the band, the isoelectric point was calculated using the calibration curve. FIG. 12 shows the change in isoelectric point with respect to each sample.

As shown in FIG. 12, the shift toward the acidic side after 24 hours reaction was observed in the sample obtained using 3,4-DGE. Accordingly, it is understood that formation of AGE-proteins has started already after 24 hours reaction.

## 5. SDS-PAGE

The samples obtained using 3,4-DGE and MGO each were subjected to SDS-PAGE. The results are shown in the electrophoretograms in FIG. 13. FIG. 13(A) shows the result obtained using 3,4-DGE, while FIG. 13(B) shows the result obtained using MGO. The lanes show the results with respect to the molecular-weight marker, native BSA, and samples (2, 4, 8, 24, 72, and 168 hours) sequentially from the left.

As shown in the figures, since disturbance in the band is observed after 2 to 4 hours reaction, it is considered that the protein structure was changed considerably and AGEs were formed. The similar change in band also was observed with respect to MGO that was a carbonyl compound that causes known AGE formation.

<Reference Example 2>

With respect to 3,4-DGE and other carbonyl compounds, the reactivities thereof with proteins were evaluated. The carbonyl compounds used herein were 3,4-DGE, MGO, GO, 3-DG, AA, FA, Fur, 5-HMF, and Glu.

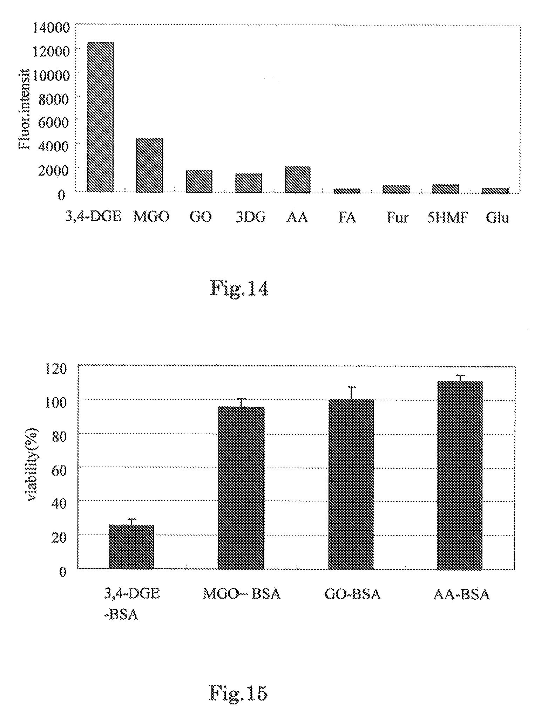

BSA was dissolved in PBS (pH 7.4) so as to be 10 mg/ml. Various carbonyl compounds each were added thereto in such a manner as to have a concentration of 30 mmol/L. Then they were allowed to react at 37°C for 24 hours. After the reaction, denaturation (AGE formation) of the BSA was evaluated according to the fluorescence intensity (at an excitation wavelength of 360 nm and a fluorescence wavelength of 430 nm) of the reaction solutions. The result is shown in FIG. 14.

As shown in FIG. 14, the BSA solution containing 3,4-DGE added thereto exhibited very strong fluorescence as compared to those containing the other carbonyl compounds. From this result, it was confirmed that 3,4-DGE was a potent mediator for AGEs that had a high reactivity to proteins.

<Reference Example 3>

The cytotoxicity of AGE-proteins derived from 3,4-DGE was determined.

In order to examine the biological activities of AGE-proteins derived from various carbonyl compounds, a cytotoxic test was carried out. The AGE-proteins used herein were 3,4-DGE-BSA, MGO-BSA, GO-BSA, and AA-BSA that had been prepared in the same manner as in Example 3.

Human peritoneal mesothelial cells (HPMCs) that had been suspended in FBS-M199 medium were seeded into a 96-well plate (manufactured by Iwaki) in an amount of 3400 cells/well and then were cultured overnight. Adherent cells in each well were exposed to M199 medium (0.1 mL/well) containing 8.4% FBS in which AGE-proteins had been dissolved in such a manner as to be 1 mg/mL, and were cultured for four days. After completion of the exposure, the medium was removed. Thereafter, the adherent cells were cultured in M199 medium containing a 10% chromogenic substrate (Trade Name: WST-1; manufactured by TAKARA BIO, INC.). Then the absorbance at a wavelength of 450 nm was measured 40 minutes later and thereby the viable cell count was determined. The result is shown in the graph in FIG. 15. In FIG. 15, "DGE-BSA" denotes "3,4-DGE-BSA".

As shown in FIG. 15, the proteins other than 3,4-DGE did not affect the cell activity. On the other hand, the activity of the cells exposed to the BSA solution in which AGEs were formed with 3,4-DGE had decreased to approximately 25%. From this result, it was confirmed that the AGE-proteins formed from 3,4-DGE showed very high cytotoxicity.

## Example 8

The formation of AGEs from 3,4-DGE contained in a peritoneal dialysate was detected using the antibody prepared in Example 1.

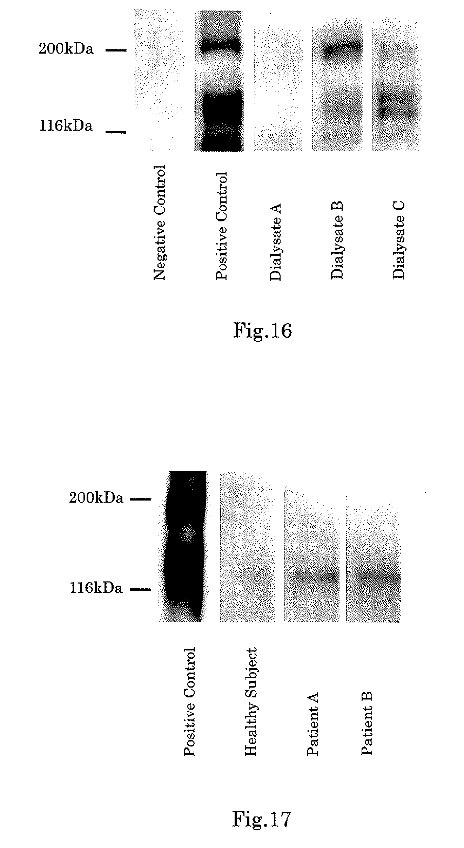

The peritoneal dialysates used herein were a dialysate A free from 3,4-DGE, a dialysate B containing 3,4-DGE whose concentration was 15 $\mu$M, and a dialysate C containing 3,4-DGE whose concentration was 6 $\mu$M. First, each dialysate and 200 mM of sodium phosphate buffer (pH 7.4) were mixed together at a volume ratio of 9:1 (v/v). Then, the pH thereof was adjusted to 7.15 to 7.27 and then HSA (human serum albumin) was dissolved therein to provide a concentration 2 mg/ml. This solution was incubated at 37°C for four weeks. Thus, assay samples were obtained. Then 3 $\mu$g of each sample was subjected to SDS-PAGE, and Western blotting was carried out in the same manner as in Example 3 using the antibody prepared in Example 1. In addition, SDS-PAGE and Western blotting were carried out in the same manner with respect to HSA and 3,4-DGE-HSA that were employed as a negative control and a positive control, respectively. In this case, 3,4-DGE-HSA used as a positive control was prepared in the same manner as in Example 3. The results are shown in FIG. 16. As shown in FIG. 16, in the assay samples obtained through incubation of HSA and each of the dialysates A, B, and C that contained 3,4-DGE, bands that indicated the reaction with the antibody were observed around 116 kDa and 200 kDa. These bands observed around 116 kDa and 200 kDa are considered to be dimers and trimers of HSA cross-linked through the reaction with 3,4-DGE since the molecular weight of HSA is 66 kDa. As described above, it was confirmed that when dialysates containing 3,4-DGE are used, AGEs derived from 3,4-DGE were formed through the reaction between the dialysates and proteins. Furthermore, since such AGEs can be detected with the antibody prepared in Example 1, it is conceivable that AGEs that have been formed also can be quantified through a comparison in color density in bands.

Example 9

Serum proteins of renal failure patients were used as assay samples and the presence of AGEs derived from 3,4-DGE was determined using the antibody prepared in Example 1.

Serum proteins (whole protein rich in albumin) of nine renal failure patients were used as samples, and 8 $\mu$g of each sample was subjected to SDS-PAGE. Then Western blotting was performed in the same manner as in Example 3 using the antibody produced in Example 1. In addition, serum protein of a healthy subject also was used as a sample. A positive control used herein was the same 3,4-DGE-HSA as in Example 8. The results are shown in FIG. 17. In FIG. 17, results with respect to two of the nine patients (Patient A and Patient B) are shown. As shown in FIG. 17, in the case of the samples of Patient A and Patient B, bands indicating the reaction with the antibody were observed around 116 kDa. Similarly, with respect to the remaining seven patients, similar results to those shown in FIG. 17 were obtained. On the other hand, bands hardly were found in the sample of the healthy subject. From these results, it was confirmed that AGEs derived from 3,4-DGE were present in the serums of renal failure patients.

Industrial Applicability

As described above, the anti-AGE antibodies of the present invention allow AGEs derived from 3,4-DGE to be detected, for example. Accordingly, it can be said that the present invention is useful for further study of the aforementioned 3,4-DGE-derived AGEs, diagnoses of various diseases that are considered to involve the 3,4-DGE-derived AGEs, etc.

**Claims**

1.  An anti-AGE antibody that is an antibody against an advanced glycation endproduct (AGE), wherein the AGE is a reaction product of 3,4-dideoxyglucosone-3-ene (3,4-DGE) and a protein or peptide.

2.  The anti-AGE antibody according to claim 1, wherein the anti-AGE antibody does not react with a reaction product of a protein or peptide and at least one carbonyl compound selected from the group consisting of methylglyoxal (MGO), glyoxal (GO), 3-deoxyglucosone (3-DG), 5-hydroxymethyl-furfural (5-HMF), furfural (Fur), formaldehyde (FA), glucose (Glu), and acetaldehyde (AA).

3.  The anti-AGE antibody according to claim 1, wherein the anti-AGE antibody does not react with a protein or peptide that has at least one of a pentosidine residue and a carboxymethyllysine (CML) residue.

4.  The anti-AGE antibody according to claim 1, wherein the AGE is a reaction product that is obtained by incubating 3,4-DGE and a protein or peptide at 25 to 50°C.

5.  The anti-AGE antibody according to claim 1, wherein the protein is serum albumin.

6.  The anti-AGE antibody according to claim 5, wherein the serum albumin is at least one albumin selected from the group consisting of rabbit serum albumin, bovine serum albumin, and human serum albumin.

7.  The anti-AGE antibody according to claim 6, wherein the AGE is a reaction product obtained by adding 3,4-DGE to a protein in such a manner that the 3,4-DGE is 2.5-equivalent to an amino group of the protein and then incubating it at 37°C for three days, and thereafter, further adding 3,4-DGE in such a manner that the 3,4-DGE is 2.5-equivalent to an amino group of the protein and then incubating it at 37°C for four days.

8.  The anti-AGE antibody according to claim 1, wherein the antibody is a polyclonal antibody.

9.  The anti-AGE antibody according to claim 1, wherein the antibody is a monoclonal antibody.

10. The anti-AGE antibody according to claim 1, wherein the antibody is an antibody obtained by immunizing a host animal using the AGE as an antigen and then isolating it from blood or abdominal dropsy of the host animal.

11. The anti-AGE antibody according to claim 10, wherein the host animal is a rabbit.

12. A method of detecting an AGE in a sample through an antigen-antibody reaction between the AGE in the sample and an anti-AGE antibody against the AGE, the antigen-antibody reaction being caused by allowing the sample to react with the anti-AGE antibody,

wherein the AGE is a reaction product of 3,4-DGE and a protein or peptide, while the anti-AGE antibody is an anti-AGE antibody according to claim 1.

13. The method of detecting an AGE according to claim 12, wherein the antigen-antibody reaction is detected by at least one immunological method selected from the group consisting of an enzyme immunoassay, a radioimmunoassay, a latex agglutination method, and a gold colloid particle method.

14. A method of detecting a carbonyl compound in a sample that forms an AGE, using an anti-AGE antibody against the AGE,

wherein the carbonyl compound is 3,4-DGE, the AGE is a reaction product of the 3,4-DGE and a protein or peptide, and the anti-AGE antibody is an anti-AGE antibody according to claim 1, and the method comprises:

allowing the 3,4-DGE in the sample and the protein or peptide to react with each other;

allowing a product obtained through the reaction and the anti-AGE antibody to react with each other;

detecting an AGE formed through an antigen-antibody reaction between the product and the anti-AGE antibody; and

qualitatively or quantitatively determining the 3,4-DGE in the sample from the presence or amount of the AGE.

15. The method of detecting a carbonyl compound according to claim 14,

wherein the antigen-antibody reaction is detected by at least one immunological method selected from the group consisting of an enzyme immunoassay, a radioimmunoassay, a latex agglutination method, and a gold colloid particle method.

16. The method of detecting a carbonyl compound according to claim 14,

wherein the sample is a dialysate, an intravenous drip, an injection, a foodstuff, or a body fluid.

17. An immunoreagent comprising an anti-AGE antibody against an AGE,

wherein the AGE is a reaction product of 3,4-DGE and a protein or peptide, while the anti-AGE antibody is an anti-AGE antibody according to claim 1.

18. The immunoreagent according to claim 17, wherein the immunoreagent is a reagent to be used for detecting the AGE that is a reaction product of 3,4-DGE and a protein or peptide.

19. The immunoreagent according to claim 17, wherein the immunoreagent is a reagent to be used for detecting the 3,4-DGE.

20. A method of producing an anti-AGE antibody that is an antibody against an advanced glycation endproduct (AGE),

wherein the AGE is a reaction product of 3,4-dideoxyglucosone-3-ene (3,4-DGE) and a protein or peptide, and

the method comprises immunizing a host animal other than a human using the AGE as an antigen and recovering an antibody against the AGE formed through the immunizing.

21. The method of producing an anti-AGE antibody according to claim 20, further comprising forming the AGE by incubating 3,4-DGE and a protein or peptide at 25 to 50°C.

22. The method of producing an anti-AGE antibody according to claim 20, wherein the protein is serum albumin.

23. The method of producing an anti-AGE antibody according to claim 22, wherein the serum albumin is at least one albumin selected from the group consisting of rabbit serum albumin, bovine serum albumin, and human serum albumin.

24. The method of producing an anti-AGE antibody according to claim 20, wherein the AGE is formed by adding 3,4-DGE to a protein in such a manner that the 3,4-DGE is 2.5-equivalent to an amino group of the protein and then incubating it at 37°C for three days, and thereafter, further adding 3,4-DGE in such a manner that the 3,4-DGE is 2.5-equivalent to an amino group of the protein and then incubating it at 37°C for four days.

25. The method of producing an anti-AGE antibody according to claim 20, wherein the process of recovering is a process of isolating the antibody from blood or abdominal dropsy of the host animal that has been immunized.

26. The method of producing an anti-AGE antibody according to claim 20, wherein the host animal is a rabbit.

Fig.1

(A)

Competitive Inhibitor (µg/ml)

- 3,4-DGE-RSA
- RSA
- BSA
- HSA
- glycated HSA

(B)

Competitive Inhibitor (µg/ml)

- 3,4-DGE-RSA
- MGO-BSA
- GO-BSA
- 3-DG-BSA

Fig.2

(1)3,4-DGE-BSA    (9)3-DG-BSA
(2)BSA    (10)5-HMF-BSA
(3)3,4-DGE-RSA    (11)Fur-BSA
(4)RSA    (12)AA-BSA
(5)3,4-DGE-HAS    (13)FA-BSA
(6)HAS    (14)glycer-BSA
(7)MGO-BSA    (15)glycol-BSA
(8)GO-BSA    (16)Glu-BSA

(1) (2) (3) (4) (5) (6) (7) (8) (9) (10) (11) (12) (13) (14) (15) (16)

Lane No.

Fig.3

(a) Not Exposed to 3,4-DGE (Control)

(b) Exposed to 30 μM 3,4-DGE

(c) Exposed to 250 μM 3,4-DGE

Fig.4

Fig.5

(A)

(B)

Fig.6

Fig.7

Fig.8

Fig.9

Fig.10

(A) Residual Ratio of Arg Residues (%)

(B) Residual Ratio of Lys Residues (%)

Fig.11

Fig.12

Fig.13

Fig.14

Fig.15

200kDa —

116kDa —

Negative Control

Positive Control

Dialysate A

Dialysate B

Dialysate C

Fig.16

200kDa —

116kDa —

Positive Control

Healthy Subject

Patient A

Patient B

Fig.17

**INTERNATIONAL SEARCH REPORT**

| International application No. |
| --- |
| PCT/JP2006/306906 |

A. CLASSIFICATION OF SUBJECT MATTER
*C07K16/18*(2006.01), *C07K16/44*(2006.01), *C07K1/22*(2006.01), *C12P21/08* (2006.01), *G01N33/53*(2006.01), *G01N33/577*(2006.01)

According to International Patent Classification (IPC) or to both national classification and IPC

B. FIELDS SEARCHED

Minimum documentation searched (classification system followed by classification symbols)
C07K16/00-16/44, C07K1/22, C12P21/08, G01N33/53, G01N33/577

Documentation searched other than minimum documentation to the extent that such documents are included in the fields searched

Electronic data base consulted during the international search (name of data base and, where practicable, search terms used)
JSTPlus(JDream2), JMEDPlus(JDream2), Igaku･Yakugaku Yokoshu Zenbun Database, PubMed

C. DOCUMENTS CONSIDERED TO BE RELEVANT

| Category* | Citation of document, with indication, where appropriate, of the relevant passages | Relevant to claim No. |
| --- | --- | --- |
| Y | Takashi YAMAMOTO et al., "3,4DGE no Seisei Yoin to Kagakuteki Hannosei", Journal of Japanese Society for Dialysis Therapy, 18 May, 2004 (18.05.04), Vol.37, Supplement, page 890 (O-0979) | 1-26 |
| Y | Masayoshi TAKEUCHI et al., "CAPD Ekichu GDPs to ni Yurai suru AGEs no Seisei Keiro no Sokatsu to Seibutsu Sayo", Fukumaku Toseki 2004, 31 July, 2004 (31.07.04), Jin to Toseki, Vol.57, separate volume 2004, pages 546 to 549 | 1-16,20-26 |
| Y | TAKEUCHI, Masayoshi et al., Immunological detection of a novel advanced glycation end-product., Molecular Medicine, 2001, Vol.7, No.11, pages 783 to 791. | 2-4,7,14-16, 20-26 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 27 April, 2006 (27.04.06) | 16 May, 2006 (16.05.06) |

| Name and mailing address of the ISA/ Japanese Patent Office | Authorized officer |
| --- | --- |
| Facsimile No. | Telephone No. |

Form PCT/ISA/210 (second sheet) (April 2005)

**INTERNATIONAL SEARCH REPORT**

International application No.

PCT/JP2006/306906

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2004-155753 A (Toray Industries, Inc.), 03 June, 2004 (03.06.04) (Family: none) | 2-4,7,14-26 |
| Y | JP 2004-323515 A (Sanwa Kagaku Kenkyusho Co., Ltd.), 18 November, 2004 (18.11.04) (Family: none) | 2-4,7,14-26 |
| Y | Edited by The Japanese Biochemical Society, "Shin Seikagaku Jikken Koza 12 Bunshi Men'eki gaku III Kogen· Kotai· Hotai", Tokyo Kagaku Dojin, 05 February, 1992 (05.02.92), first edition, pages 1 to 2, 99 to 101 | 12-16,20-26 |

Form PCT/ISA/210 (continuation of second sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 9178740 A

- JP 11246599 A


**Non-patent literature cited in the description**

- **SCHLEICHER, E. D et al.** *J. Clin. Invest,* 1997, vol. 99, 457
- **SANAKA, T et al.** *Nephron,* 2002, vol. 91, 64
- **OBAYASHI, H et al.** *Biochem. Biophys. Res Commun.,* 1996, vol. 226, 37
- **MIYATA, S ; MONNIER, V.** *J. Clin. Invest,* 1992, vol. 89, 1102
- **HAYASE, F et al.** *J. Biol. Chem.,* 1989, vol. 263, 3758
- **TAKEUCHI, MASAYOSHI et al.** Nippon Rinsho. Nippon Rinsho, 2002, vol. 60
- **TAKEUCHI, M et al.** *Molecular Medicine,* 2001, vol. 7, 783